# EUROPEAN PATENT APPLICATION

(11) **EP 3 222 238 A1**
(43) Date of publication of application: **27.09.2017**
(21) Application number: 15860490.0
(22) Date of filing: 09.11.2015
(51) Int. Cl.: A61B 18/12

(54) **HIGH-FREQUENCY FORCEPS**

(30) Priority: 19.11.2014 JP 2014234368
(71) Applicant: Kyushu University, National University Corporation, Fukuoka-shi, Fukuoka 812-8581 (JP); Hogy Medical Co., Ltd., Tokyo 107-8615 (JP)
(72) Inventor: NAKADATE, Ryu, Fukuoka-shi Fukuoka 812-8581 (JP); HASHIZUME, Makoto, Fukuoka-shi Fukuoka 812-8581 (JP); NAGAI, Shunsuke, Tokyo 107-8615 (JP); FUJITA, Hiroyasu, Tokyo 107-8615 (JP); KATO, Jiro, Tokyo 107-8615 (JP)
(74) Representative: Viering, Jentschura & Partner mbB Patent- und Rechtsanwälte
(86) International application number: PCT/JP2015/081448
(87) International publication number: WO 2016/080223

(57) **Abstract**

To provide a treatment instrument that combines a capability to grip a target tissue and a capability to resect and ablate the target tissue without the need to interchange left and right devices or adjust a field of view of an endoscope, which can reduce burden on a surgeon.

A high frequency forceps includes a pair of forceps pieces configured to open and close on a pivot and equipped with incision blades adapted to pass a high-frequency current to a living tissue, in which the incision blades are formed, respectively, on opposite faces of the pair of forceps pieces, extending from a side of the pivot to a distal side; and the incision blades are spaced away from each other when the pair of forceps pieces is closed.

## Description

### Technical Field

The present invention relates to a high frequency forceps as an endoscopic treatment instrument inserted into a flexible endoscope, and specifically, to a high frequency forceps having a function of a high frequency scalpel and forceps that aims to be inserted into a treatment instrument channel of a flexible endoscope or a treatment instrument passage tube attached to a flexible endoscope, is caused to reach an abdominal organ such as the stomach or intestines together with the flexible endoscope from the mouth or anus, and used for resecting cancer such as epithelial cancer.

### Background Art

Recent years, operative methods such as endoscopic submucosal dissection (ESD) have come to be used, where ESD involves inserting a treatment instrument through the mouth or anus and removing one slice from an upper layer of a mucous membrane over a wide area of the stomach or large intestine without relying on a laparotomy or endoscopic surgery. Furthermore, an operative method (NOTES: Natural Orifice Translumenal Endoscopic Surgery) is known that involves inserting a flexible endoscope such as a gastric or large intestine camera through the mouth, anus, vagina, or urethra that originally exists in the surface of the body, then taking the flexible endoscope to an abdominal cavity by penetrating a stomach or large-intestine wall, and conducting diagnosis or treatment on an abdominal organ.

Since the natural orifice translumenal endoscopic surgery typified by endoscopic submucosal dissection (ESD) conducts treatment or the like by inserting a treatment instrument such as a forceps or a scalpel together with a flexible endoscope through the mouth or the like that originally exists in the surface of the body, and taking the treatment instrument to a diseased part, the surgery causes no damage to the surface of the body, can reduce the risk of complications such as infection or adhesion of the abdominal wall, which accompany ordinary surgery, and can reduce invasion into the human body.

As described in Patent Literature 1, the treatment instrument used for the natural orifice translumenal endoscopic surgery includes a bending portion inserted into the flexible endoscope and used to bendably manipulate the treatment instrument projecting from a distal end of the flexible endoscope. Also, the treatment instrument includes a sheath wire adapted to transmit bending motion to the bending portion and an operating portion used to manipulate the bending motion of the bending portion by pushing and pulling the sheath wire.

Also, regarding configurations of treatment instruments, for example, a configuration is known in which a diseased part is resected or ablated by passing a high-frequency current through a forceps such as described in Patent Literature 1 or a rod-shaped needle knife such as described in Patent Literature 2.

With this configuration, in resecting or ablating a diseased part with a needle knife by gripping the diseased part with the forceps, the diseased part can be resected or ablated by placing an incision blade gripped with the forceps or the like in contact with the diseased part, and moving the incision blade horizontally, and thereby continuing to cut the diseased part.

### Citation List

### Patent Literature

Patent Literature 1: Japanese Patent Laid-Open No. 2010-511440
Patent Literature 2: Japanese Patent Laid-Open No. 2010-42155

### Summary of Invention

### Technical Problem

However, regarding methods for resecting a diseased part using the treatment instruments described in Patent Literatures 1 and 2, a method is known that first injects saline or the like under a target tissue to be resected from the diseased part using a treatment instrument equipped with a needle, causing the target tissue to float up from other tissues such as an underlying submucosal layer and then incises the target tissue over an entire circumference thereof using a treatment instrument equipped with a high frequency knife. After incision of the entire circumference, the target tissue is pulled upward using another treatment instrument such as a forceps and then an underlayer of the target tissue is cauterized and ablated.

Since the treatment instruments described in Patent Literatures 1 and 2 grip, incise, and ablate a target part using a forceps and a high frequency knife in this way, there is a problem in that, for example, when making a circumferential incision clockwise if a surgeon wants to continue the incision counterclockwise, it becomes necessary to replace the high frequency knife with the forceps, reverse the endoscope itself, and extend surgical time, increasing complexity of maneuvering for the surgeon. Furthermore, when the high frequency knife is located outside a field of view of a camera, if the high frequency knife inadvertently touches something other than the target tissue, there is danger that a tissue other than the target tissue will be resected.

The present invention has been made to solve the above problem and specifically has an object to provide a treatment instrument that combines a capability to grip a target tissue and a capability to resect and ablate the target tissue without the need to interchange left and right devices or adjust a field of view of an endoscope, which can reduce burden on a surgeon.

### Solution to Problem

To solve the above problem, the present invention provides a high frequency forceps including a pair of forceps pieces configured to open and close on a pivot and equipped with incision blades adapted to pass a high-frequency current to a living tissue, wherein the incision blades are formed, respectively, on opposite faces of the pair of forceps pieces, extending from a side of the pivot to a distal side; and the incision blades are spaced away from each other when the pair of forceps pieces is closed.

Preferably in the high frequency forceps according to the present invention, distal portions of the pair of forceps pieces are provided with abutting portions configured to abut each other when the pair of forceps pieces is closed.

Also, preferably in the high frequency forceps according to the present invention, the abutting portions come substantially into point contact with each other.

Also, preferably in the high frequency forceps according to the present invention, the incision blades have an approximately triangular shape in a section orthogonal to an extending direction of the pair of forceps pieces.

Also, preferably in the high frequency forceps according to the present invention, the incision blades are subjected to insulation treatment except for vertices of the triangular shape.

### Advantageous Effects of Invention

According to the present invention, since the forceps pieces equipped with the respective incision blades are formed on opposite sides so as to extend from the side of the pivot to the distal side and the incision blades are spaced away from each other when the pair of forceps pieces is closed, by passing a high-frequency current through the incision blades while gripping a target tissue in the pair of forceps pieces, the target tissue existing between the incision blades can be resected and ablated.

Also, according to the present invention, since the abutting portions configured to abut each other when the pair of forceps pieces is closed are provided, the target tissue can be gripped securely.

Also, according to the present invention, since the abutting portions are configured to come substantially into point contact with each other, the target tissue resected by the high-frequency current is prevented from being burnt and adhering onto the forceps pieces, and opening and closing action of the forceps is not obstructed.

Also, according to the present invention, since each forceps piece has an approximately triangular sectional shape, the high-frequency current can be passed intensively through electrodes, improving cutting capacity of the knife and allowing the target tissue to be incised while reducing unnecessary damage to surrounding tissues.

Also, according to the present invention, since the incision blades are subjected to insulation treatment except for the vertices of the triangular shape, the cutting capacity can be improved by passing the high-frequency current through the electrodes more intensively.

### Brief Description of Drawings

[FIG. 1] FIG. 1 is a perspective view of a high frequency forceps according to the present embodiment.
[FIG. 2] FIG. 2 is an enlarged view of a distal end of the high frequency forceps according to the present embodiment.
[FIG. 3] FIG. 3 is a sectional view taken along line A-A in FIG. 2.
[FIG. 4] FIGS. 4(a) and 4(b) are diagrams for illustrating conditions in which the high frequency forceps according to the present embodiment is used.
[FIG. 5] FIG. 5 is a diagram for illustrating a condition in which the high frequency forceps according to the present embodiment is used.
[FIG. 6] FIG. 6 is a perspective view showing how a target tissue is gripped.
[FIG. 7] FIG. 7 is a top view showing how a target tissue is gripped.
[FIG. 8] FIG. 8 is a perspective view showing a variation of the high frequency forceps according to the present embodiment.
[FIG. 9] FIG. 9 is a perspective view showing a variation of the high frequency forceps according to the present embodiment.

### Description of Embodiment

A high frequency forceps according to the present invention will be described below with reference to the drawings. Note that the embodiment described below is not intended to limit the claimed invention and that a combination of all the features described in the embodiment is not necessarily essential for the means to solve the problems according to the present invention.

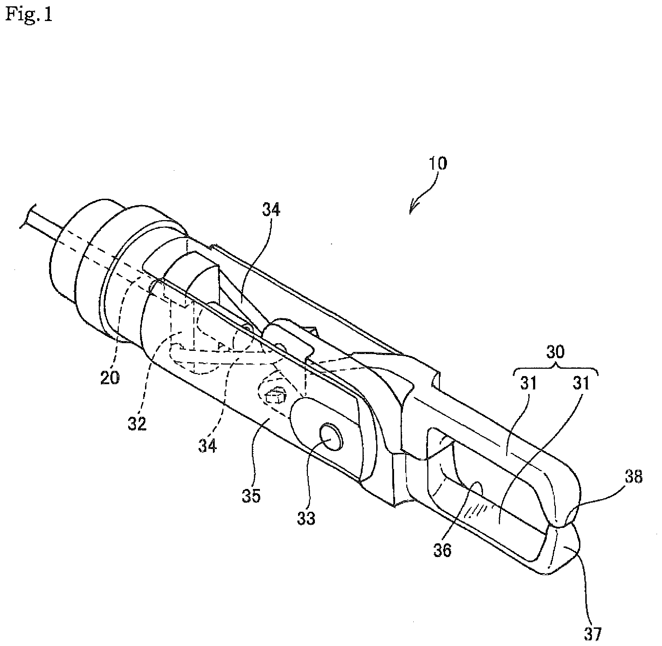

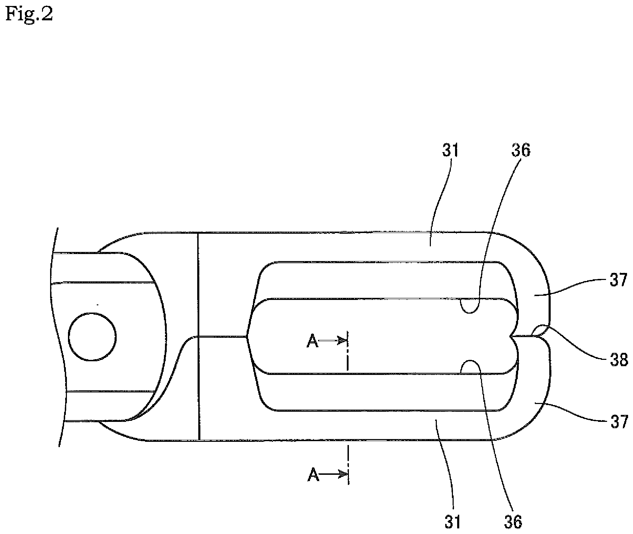

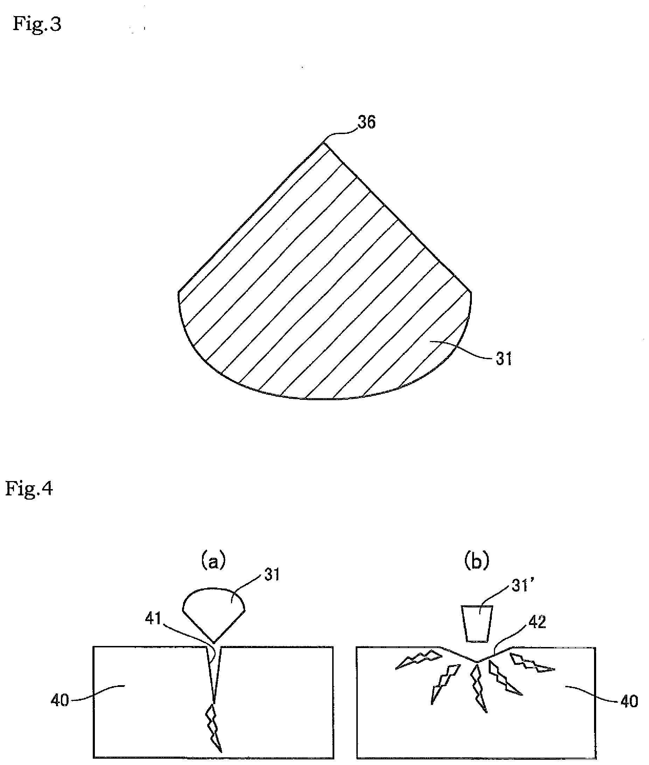

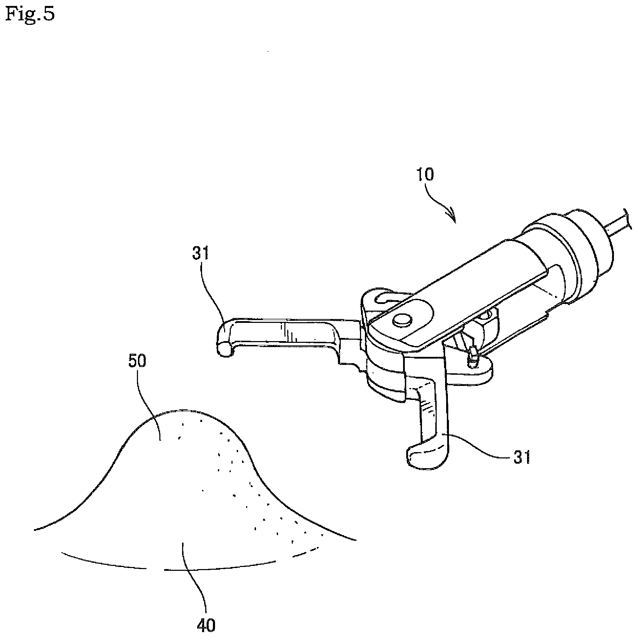

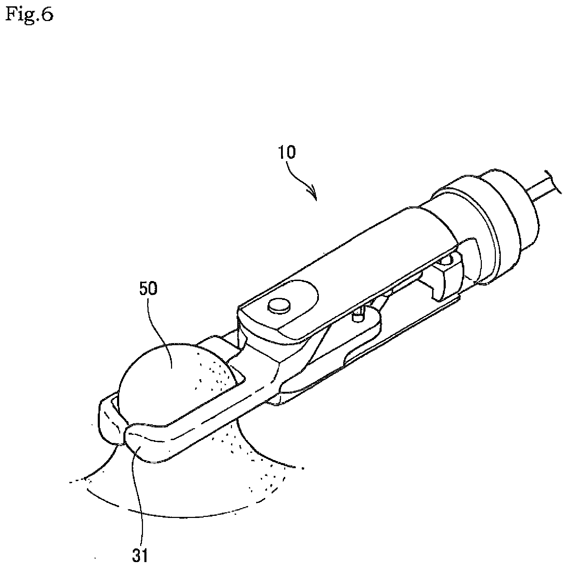

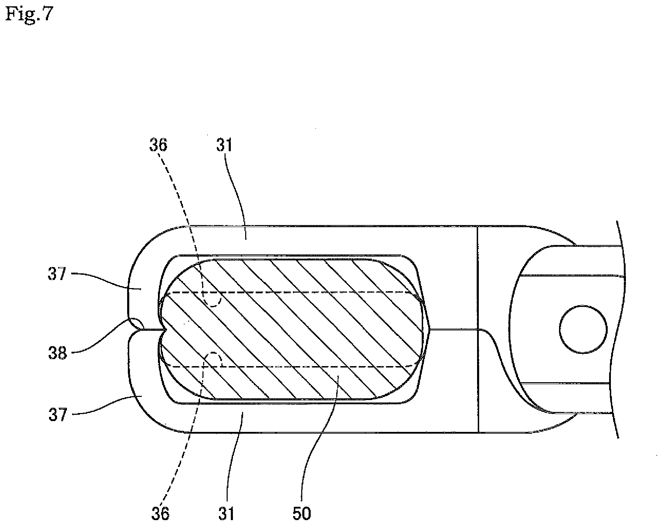

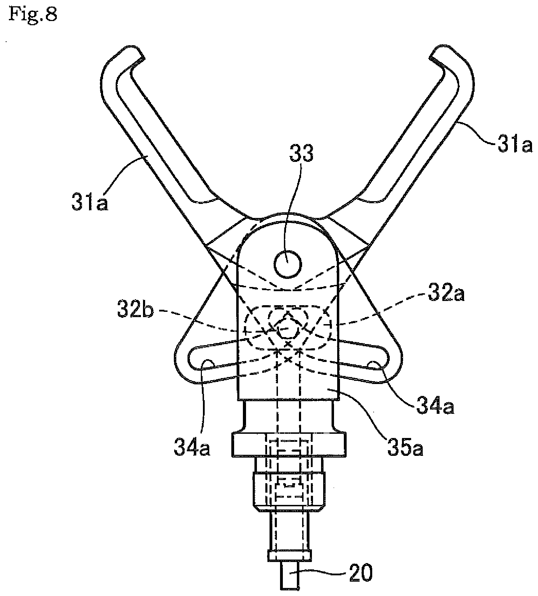

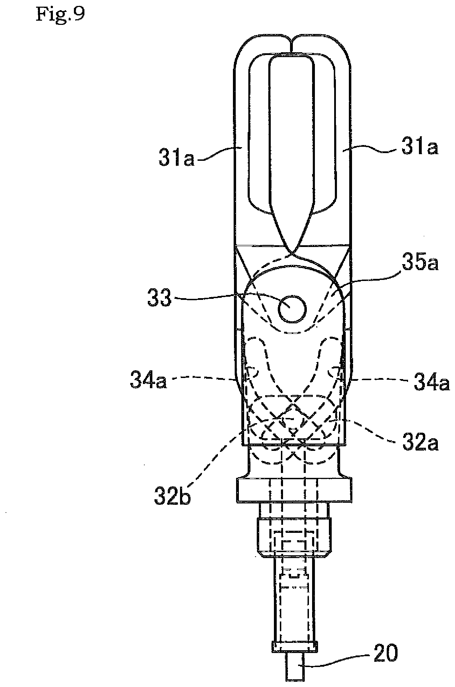

FIG. 1 is a perspective view of a high frequency forceps according to the present embodiment, FIG. 2 is an enlarged view of a distal end of the high frequency forceps according to the present embodiment, FIG. 3 is a sectional view taken along line A-A in FIG. 2, FIGS. 4(a) and 4(b) are diagrams for illustrating conditions in which the high frequency forceps according to the present embodiment is used, FIG. 5 is a diagram for illustrating a condition in which the high frequency forceps according to the present embodiment is used, FIG. 6 is a perspective view showing how a target tissue is gripped, FIG. 7 is a top view showing how a target tissue is gripped, and FIGS. 8 and 9 are perspective views showing variations of the high frequency forceps according to the present embodiment.

As shown in FIG. 1, the high frequency forceps 10 according to the present embodiment performs opening and closing action when a forceps 30 made up of a pair of forceps pieces 31 pivot with respect to each other on a pin 33 serving as a pivot. The forceps pieces 31 are equipped with opening and closing wires 34 intersecting each other by being attached to a proximal side, and the opening and closing wires 34 are connected to a movable body 32 adapted to move in response to push-pull action of a device wire 20 connected to a non-illustrated operating portion mounted on the proximal side. Note that the movable body 32 and the opening and closing wires 34 are contained in a forceps base 35.

The device wire 20 is connected to the operating portion described above by being inserted in a non-illustrated sheath attached to one end of the forceps base 35. Note that the sheath is configured to be bendable along with bending of the endoscope so as not to obstruct bending motion of the endoscope.

Also, as shown in FIG. 2, each forceps piece 31 is formed of a conductive metal and a distal portion 37 is formed on the distal side of the forceps piece 31, bending toward the counterpart forceps piece 31 opposed to the given forceps piece 31. Besides, an abutting portion 38 is formed on that face of the distal portion 37 that abuts the distal portion 37 of the counterpart forceps piece 31. Furthermore, an angled tip bent inward is formed in the distal portion 37 to prevent a tissue gripped between the pair of forceps pieces 31 from falling off. In FIG. 2, preferably size of the pair of forceps pieces 31 in a width direction is 2.8 mm or less so that the pair of forceps pieces 31 can pass through an endoscope channel, and more preferably 2.3 mm or less so that the pair of forceps pieces 31 can pass through the bent endoscope channel without much resistance.

The abutting portions 38 are formed to reduce contact area in order to prevent burn-in of a target tissue when a high-frequency current flows, and ideally it is preferable to minimize the contact area to realize point contact.

Furthermore, since the distal portion 37 is bent toward the other forceps piece 31, when the pair of forceps pieces 31 is closed, the abutting portions 38 abut each other, forming a gap between the forceps pieces 31.

As the gap is formed between the pair of forceps pieces 31 in this way, even if the high frequency forceps 10 according to the present embodiment is used continuously for a long time, it is possible to prevent the target tissue and surrounding tissues from being burned and stuck to the forceps pieces 31 as adherents and thereby prevent the forceps 30 from becoming unopenable.

Note that as a base material of the forceps pieces 31, any material may be used as long as the high-frequency current can be passed through the incision blades 36, but for example, stainless steel, iron, copper, aluminum, tungsten, silver, glass, or the like can be used suitably. Also, if ceramic, polyetheretherketone (PEEK), polycarbonate (PC), amorphous thermoplastic polyetherimide (PEI), or the like is used as a base material, the incision blades 36 may be constructed by combining electrodes configured to pass a high-frequency current. Regarding the gap between the pair of the forceps pieces 31, a large gap can prevent resected tissues and the like from burning on, but too large a gap reduces strength, and thus preferably size of the gap is 0.7 to 1.0 mm.

Furthermore, the incision blades 36 are formed on the opposite faces of the forceps pieces 31, extending from the pin 33 to the distal side. Also, to make the incision blades 36 as thin as possible, in the high frequency forceps 10 according to the present embodiment, as shown in FIG. 3, each forceps piece 31 has an approximately triangular shape in a section orthogonal to an extending direction of the forceps piece 31, with a vertex of the triangular shape being located on the incision blade 36. Also, regarding an angle at a cutting edge, the acuter the angle, the easier it is to machine the incision blades 36 finely, but the acuter the angle, the lower the strength becomes, and thus preferably the angle at the cutting edge is about 80 to 100 degrees.

Furthermore, the forceps pieces 31 are subjected to insulation treatment except for the incision blades 36. Note that any type of insulation treatment may be applied as long as high-frequency current is kept from passing, but, for example, fluorocarbon resin, ceramic, polyolefin, natural rubber, nitrile rubber, or the like can be used suitably. Such an insulation treatment, when applied, can prevent resected tissues from being carbonized and attached as contamination to the incision blades 36, sliding portions of the forceps pieces 31, and a neighborhood of the pin 33 and obstructing operation of the high frequency forceps 10. Incidentally, coating may be used as the insulation treatment, but alternatively the forceps pieces 31 themselves may be made of an insulator or electrodes made of a conductive metal may be fitted as incision blades 36 in the distal ends of forceps pieces 31.

With a conventional high frequency knife 31' having an obtuse angle such as shown in FIG. 4(b), high energy output is needed in order to obtain a required cutting capacity because of large electrode area, while at the same time there is a risk of causing unnecessary damage to surrounding tissues because of a large cut area 42 due to diffusion of a high-frequency current. In contrast, with the high frequency forceps 10 according to the present embodiment, since the forceps pieces 31 are subjected to insulation treatment except for the incision blades 36, causing the high-frequency current passing through the forceps pieces 31 to be applied intensively to a target tissue 40 as shown in FIG. 4(a)*, thereby allowing a cut area 4 to be formed using less current, preventing diffusion of the high-frequency current, and thereby curbing unnecessary damage to surrounding tissues, the cutting capacity is improved.

Next, a method for using the high frequency forceps 10 according to the present embodiment will be described with reference to FIGS. 5 to 7. The following description assumes that the gastric mucosa is resected by endoscopic surgery.

First an insertion portion of the endoscope is inserted into the body cavity of a patient, and the distal end of the insertion portion is moved to a neighborhood of a diseased part 50, which is an object to be treated.

The high frequency forceps 10 according to the present embodiment is inserted into the endoscope channel with the forceps 30 closed, and is held with the forceps 30 projecting from the distal end of the insertion portion of the endoscope. In this state, the surgeon brings the forceps 30 close to the diseased part 50 with the forceps 30 opened while watching a video from the endoscope and closes the forceps 30 to grip the diseased part 50 with the forceps pieces 31 as shown in FIG. 6.

In so doing, since the diseased part 50 is gripped in the gap between the pair of forceps pieces 31 as shown in FIG. 7, when a high-frequency current is passed through the forceps pieces 31, the high-frequency current flows from the incision blades 36 to a return electrode placed on the body surface thereby allowing the diseased part 50 to be resected.

In so doing, by making the incision blades 36 thin, it is possible to prevent diffusion of the high-frequency current in the diseased part 50 and thereby prevent the diseased part 50 from being damaged more than necessary.

Note that after resecting the diseased part 50, because the resected diseased part 50 can be picked up, for example, with the abutting portions 38 and extracted out of the body, surgery can be performed smoothly without carrying out the task of replacing the treatment instrument with another one equipped with forceps.

Whereas a preferred embodiment of the present invention has been described above, the technical scope of the present invention is not limited to the description of the above embodiment. Various changes or improvements can be made to the above embodiment.

Whereas description has been given of a case in which the high frequency forceps according to the present embodiment is a flexible forceps inserted into the endoscope channel of an endoscope and configured to bend along with the bending of the endoscope, by interposing, for example, plural flexible hinges in the sheath of the high frequency forceps according to the present embodiment, the direction of the forceps projecting from the endoscope channel may be configured to be freely changeable. Regarding the mode of passing a high-frequency current, whereas description has been given of a case in which a so-called monopolar mode is applied, whereby a high-frequency current is passed from the incision blades to the return electrode placed on the body surface, a so-called bipolar mode may be adopted in which a high-frequency current is passed from the incision blade of one of the pair of forceps pieces to the incision blade of the other forceps piece.

Also, whereas it has been stated that the high frequency forceps according to the present embodiment is equipped with the opening and closing wires intersecting each other by being attached to the proximal side of the forceps pieces, where the opening and closing wires are connected to a movable body adapted to move in response to the push-pull action of the device wire connected to a non-illustrated operating portion mounted on the proximal side thereby making up an opening and closing mechanism adapted to open and close the forceps pieces, the opening and closing mechanism is not limited to this form.

For example, as shown in FIGS. 8 and 9, bent grooves 34a may be formed on the proximal side of a pair of forceps pieces 31a, intersecting each other, placed line-symmetrically with respect to a longitudinal direction, intersecting each other, and configured to get engaged with engaging pins 32b formed on a movable body 32a. When the forceps pieces 31a are closed, the engaging pins 32b are engaged with the proximal side of the grooves 34a as shown in FIG. 9, and when the device wire 20 is manipulated so as to push out the movable body 32a, the engaging pins 32b move within the grooves 34a and get placed on the distal side, allowing the forceps pieces 31a to be put in an open state as shown in FIG. 8. If the opening and closing mechanism is configured in this way, an overall length of a forceps base 35a can be reduced, making it possible to downsize the high frequency forceps. It will be apparent from the description of the appended claims that any form resulting from such changes or improvements may also be included in the technical scope of the present invention.

### Reference Signs List

- 10: high frequency forceps
- 20: device wire
- 30: forceps
- 31, 31a: forceps piece
- 32, 32a: movable body
- 33: pin
- 34: opening and closing wire
- 34a: groove
- 34b: engaging pin
- 35, 35a: forceps base
- 36: incision blade
- 37: distal portion
- 38: abutting portion
- 40: target tissue
- 41, 42: cut area
- 50: diseased part

## Claims

1. A high frequency forceps including a pair of forceps pieces configured to open and close on a pivot and equipped with incision blades adapted to pass a high-frequency current to a living tissue, wherein the incision blades are formed, respectively, on opposite faces of the pair of forceps pieces, extending from a side of the pivot to a distal side; and the incision blades are spaced away from each other when the pair of forceps pieces is closed.

2. The high frequency forceps according to claim 1, wherein distal portions of the pair of forceps pieces are provided with abutting portions configured to abut each other when the pair of forceps pieces is closed.

3. The high frequency forceps according to claim 2, wherein the abutting portions come substantially into point contact with each other.

4. The high frequency forceps according to any one of claims 1 to 3, wherein the incision blades have an approximately triangular shape in a section orthogonal to an extending direction of the pair of forceps pieces.

5. The high frequency forceps according to claim 4, wherein the incision blades are subjected to insulation treatment except for vertices of the triangular shape.
